# EUROPEAN PATENT APPLICATION

(11) **EP 2 666 486 A1**
(43) Date of publication of application: **27.11.2013**
(21) Application number: 12169476.4
(22) Date of filing: 25.05.2012
(51) Int. Cl.: A61L 27/52, C12N 5/00, G01N 33/50, C08F 220/56, C08F 222/38

(54) **Micro-engineered hydrogels**

(71) Applicant: Université de Mons, 7000 Mons (BE)
(72) Inventor: Grevesse, Thomas, 7000 Mons (BE); Gabriele, Sylvain, 7000 Mons (BE)
(74) Representative: Pronovem

(57) **Abstract**

A polyacrylamide hydrogel comprising copolymerized acrylamide, bisacrylamide and N-hydroxyethylacrylamide.

## Description

### Field of the invention

The Present invention is related to functionalized polyacrylamide hydrogels to the method for producing them and to their use for *in vitro* culture of cells and *in vitro* differentiation of stem cells.

Cells perceive their microenvironment through soluble cues (growth factors, metabolites and dissolved gases), but also through insoluble cues related to the heterogeneous physical, chemical and mechanical properties of the extracellular matrix (ECM). By mechanotransduction systems, adherent cells translate external forces and ECM stimuli into a cascade of chemical and molecular events controlling multiple aspects of cell behaviour, including proliferation, differentiation, migration, gene expression.

Conversely, defective mechanotransduction are implicated in a diverse group of diseases, ranging from muscular dystrophies and dilated cardiomyopathy to metastasis. Many *in vitro* observations in modern cell biology have been performed on rigid plastic dishes and glass coverslips, often coated with a thin layer of ECM proteins or synthetic peptides containing the fibronectin-derived RGD sequence. However, such basic culture substrates do not recapitulate the whole physico-chemical complexity of the ECM and thus do not provide an acurate model for cell biology assays and especially cellular mechanosensing. Consequently, research has expanded to diverse cellular substrates in order to investigate the role of the physico-chemical properties of the ECM on cellular functions and mechanotransduction signaling pathways.

Developments in the use of matrix-functionalized synthetic substrates have enabled studies that suggested that substrate stiffness could interfere in a broad range of cellular functions.

The mechanisms by which cells sense stiffness remains poorly defined, but early evidence suggests that the sensing system involves modulation of integrins, focal adhesions and myosin-based contractility, which are also key players of other mechanotransduction pathways.

ECM not only displays a wide range of stiffnesses and several classes of proteins but also presents various densities of adhesive ligands that are important to anchorage-dependent cells.

In addition to a variety of stiffnesses, proteins and cell-ligand densities, the cell microenvironment imposes a confined adhesiveness that influence not only cell architecture and mechanics, but also cell shape, polarity and functions.

However, under classic cell culture conditions using homogeneous coated surfaces, the entirety of this spatial information are lost.

A major interest is therefore to decouple the effects of ECM stiffness, nature of protein, cell-ligand density and confined adhesiveness.

### State of the Art

To address this challenge, various methods have been proposed during the last decade, such as electron-beam, photolithography, photochemical immobilization or plasma-assisted techniques to create micropatterns of ECM proteins to constrain and control the growth of living cells on substrates with varied stiffnesses.

Although these different methods have proven very useful, most of them require technical facilities that only few biological laboratories can afford and do not permit to discriminate the influence of mechanotransduction cues.

Polyacrylamide (PAAm), originally introduced for use as a support matrix for electrophoresis, has sometimes been used as polymer-based matrix for cell biomechanics assays.

To surmount their intrinsically non-adhesive properties, PAAm surfaces can be functionalized with the heterobifunctional cross-linker N-sulfosuccinimidyl-6-[4'-azido-2'-nitrophenylamido] (sulfo-SANPAH) and ECM proteins are linked to the surface by UV activation of the sulfo-SANPAH nitrophenyl azide groups. Another method consists in treating PAAm gels with hydrazine and coupling it to proteins that have been severely oxidized by periodate.

A method based on deep UV exposure of PAAm through an optical quartz mask that requires to incubate activated PA gels with 1-ethyl-3-[3-dimethylaminopropyl]carbodiimide hydrochloride (EDC) and N-hydroxysuccinimide (NHS) water solutions prior to coat fibronectin (FN).

Despite their ability to create homogeneous and reproducible protein micropatterns on soft PA gels, these functionalization methods suffer from major limitations: long synthesis processes (e.g. dialysis, lyophilization, etc.), expensive chemical compounds (e.g. hyaluronic acid, sulfo-SANPAH, etc.), deep UV irradiation and still do not permit to modulate independently substrate stiffness, nature of protein, cell-ligand density and micropattern geometry.

### Summary of the invention

The present invention relates to a method for producing a polyacrylamide hydrogel comprising the step of mixing, in a liquid solution, monomers of acrylamide, bisacrylamide and N-hydroxyethylacrylamide and an agent causing the co-polymerization of the said monomers.

Advantageously, this method further comprises the step of fixing a biomolecule (a peptide) to the (modified-based polyacrylamide hydrogel) polymer.

Possibly, this biomolecule (peptide) is fixed by microcontact printing, for instance at a pressure of about 100 Pa to about 1 000 Pa.

Advantageously, this method further comprises the step of putting the gel in contact of a (an aqueous) solution comprising between 10 and 50 µg/ml of a biomolecule (for homogenously fixing a biomolecule).

A related aspect of the present invention is a polyacrylamide hydrogel obtainable by this method.

Therefore, the present invention relates to a polyacrylamide hydrogel comprising co-polymerized acrylamide, bisacrylamide and N-hydroxyethylacrylamide, wherein the wt ratio of bisacrylamide to acrylamide is comprised between 1% and 15% and the wt ratio of N-hydroxyethylacrylamide to acrylamide is comprised between 20% and 50%.

Preferably, this polyacrylamide hydrogel comprises between 1% (w:w) and 10% (w:w) of acrylamide

Advantageousely, this polyacrylamide hydrogel, has a stiffness comprised between 0.1 kPa and 500 kPa, preferably between 1 kPa and 100 kPa, more preferably between 2 kPa and 50 kPa and still more preferably between 5 kPa and 20 kPa.

Preferably, this polyacrylamide hydrogel further comprises a first biomolecule (peptide) fixed at the surface of this polyacrylamide hydrogel.

Preferably, this biomolecule (peptide) is a protein selected from the group consisting of albumin, laminin, fibronectin, collagen I, collagen IV, streptavidin, tenascin, reelin, cadherin, integrins, chemokines, antibodies and fragments thereof, being more preferably laminin and/or fibronectin.

Preferably, this first biomolecule (peptide) is fixed to the surface of this hydrogel from a solution comprising between 1 µg/ml and 100 µg/ml (preferably between 10 and 50, more preferably of about 20) of this first biomolecule.

Possibly, this first biomolecule(s) is (are) uniformly fixed to this polyacrylamide hydrogel.

Alternatively (preferably), this first biomolecule is heterogeneously fixed to this polyacrylamide hydrogel.

Advantageously, this first biomolecule(s) is (are) fixed on surface of between about 500 µm² and between about 5000 µm² (on this polyacrylamide hydrogel).

Advantageously, this polyacrylamide hydrogel further comprises a second biomolecule (preferably a protein, more preferably albumin) homogenously fixed to the said polyacrylamide hydrogel.

Advantageously, this polyacrylamide hydrogel further comprises animal cells.

A related aspect of the present invention is the use of this polyacrylamide hydrogel comprising animal cells as biosensor.

Another related aspect of the present invention is the use of this polyacrylamide hydrogel (comprising animal cells) for the *in vitro* culture of animal (preferably mammalian, more preferably (non embryonic) human) cells.

Another related aspect of the present invention is the use of this polyacrylamide hydrogel for the *in vitro* differentiation of animal (preferably mammalian, more preferably human non embryonic) stem cells.

The preferred use of this polyacrylamide hydrogel, is for the *in vitro* culture and/or differentiation of neuronal cell(s) or of muscle cell(s).

### Brief description of the figures

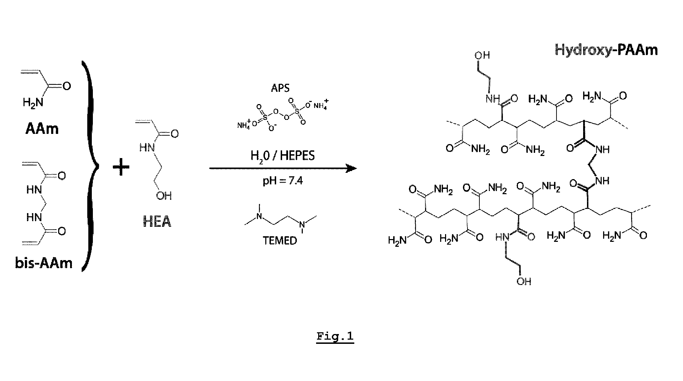

Figure 1 represents the synthesis of hydroxy-PAAm hydrogels wherein Acrylamide (AAm,), N,N'-methylenebisacrylamide (bis-AAm) and N-hydroxyethylacrylamide (HEA) are mixed together and the polymerization is initiated by adding TEMED and APS in a pH=7,4 HEPES buffer.

### Detailed description of the invention

The inventors have co-polymerized acrylamide (AAm) and bisacrylamide (bis-AAm) with N-hydroxyethylacrylamide monomers (HEA) containing a primary hydroxyl group (Figure 1) to form a hydrophilic network of polyacrylamide with hydroxyl groups (hydroxy-PAAm) by random radical polymerization.

Advantageously, the structural and mechanical properties of hydroxy-PAAm hydrogels can be finely controlled by varying the crosslinker (bisacrylamide) and/or acrylamide monomer concentration during polymerization. For instance, the inventors have succeeded in altering the network mesh size by changing the bis-AAm crosslinker concentration from 0.05 to 0.5%, while keeping the amounts of AAm and HEA monomers constant (for instance 3.2 wt % and 1.3 wt %, respectively). Conversely, the HEA content can be tailored, while keeping constant the hydrogel properties such as stiffness.

The inventors have further shown that hydroxy-PAAm hydrogels have a linear dependence of elastic moduli on cross-linker concentration from few kPa to several dozens of kPa. This range covers the whole elasticity range of natural soft tissues and represents optimal elasticities, not only for neurons (∼1 kPa) but also for muscle cells (-10 kPa). Therefore a lot, if not all, of cell types can be cultured on hydroxy-PAAm hydrogels.

Several important features are related to mechanotransduction: (i) cells are shaped by the ECM stiffness, (ii) numerous cellular functions are affected by confined adhesiveness, (iii) cellular functions and signalling pathways are dependant on the nature of ECM proteins and (iv) cell behaviours are also influenced by cell-ligand density.

The development by the inventors of bioactive micropatterns of different shapes and sizes on soft hydrogels, with a decoupled control of matrix rigidity, nature of protein and cell-ligand density represents therefore an important improvement over existing techniques.

In the context of the present invention, the term "fixed" or "stably fixed" (and/or applied, added and/or adsorbed) refers to a persistent association of the biomolecule to the (polyacrylamide) polymer hydrogels of the present invention. Preferably, this persistent association lasts (after storage at 4°C) for more than 1 week, more preferably for more than one month.

A first aspect of the present invention is therefore a method for producing a polyacrylamide (polymer) hydrogel comprising the steps of mixing, in a liquid solution, monomers of acrylamide, bisacrylamide (N,N'-Methylenebisacrylamide) and 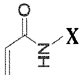 (such as N-hydroxyethylacrylamide) and a composition (or a mixture or a solution or even a pure product) causing the co-polymerization (e.g. via the production of free radicals) of these monomers, such as a composition comprising ammonium peroxide and N,N,N',N'-Tetramethylethylenediamine (TEMED) and wherein X residue is a C₁-C₆ alkyl further comprising a function selected from the group consisting of hydroxyl, carboxy, amine and aldehyde.

Preferably, this method further comprises the step of fixing (adding and/or adsorbing) a (first) biomolecule to the above described (polyacrylamide) polymer hydrogel.

Preferably, in this method, biomolecule(s) fixed (applied, added and/or adsorbed) on the (polyacrylamide) polymer hydrogels is (are) peptide(s) or protein(s), such as ECM-protein (or a fragment thereof).

Possibly, the first biomolecule(s), such as ECM-protein(s), is (are) (stably) fixed (applied, added and/or adsorbed) to predesigned regions of (flat) surfaces of the obtained (polyacrylamide) polymer hydrogel.

Preferably, in this method stamps (having a determined shape and/or geometry) were incubated with a solution comprising a first biomolecule (such as an ECM-protein solution) and these stamps were dried and placed in (conformal) contact with this (polyacrylamide) polymer hydrogel to obtain a transfer of the first biomolecule (micro)patterns on this (polyacrylamide) polymer hydrogel (allowing (stable) fixation of the biomolecule).

Possibly, (stamped) (polyacrylamide) polymer hydrogel surfaces were (further) passivated, for instance with albumin, such as bovine serum albumin (BSA), to block the non-printed surface areas.

Preferably, in this method the biomolecule(s) is (are) (stably) fixed (applied, added and/or adsorbed) by microcontact printing. For instance this microcontact printing allows resolutions comprised between about 100 nm (or 1 µm) and about 500 µm.

Preferably, in this method this microcontact printing is performed by applying a pressure comprised between about 100 Pa and about 10 000 Pa, more preferably between about 200 and about 600 Pa.

Alternatively, or in addition, (other biomolecule than the first one) biomolecule(s) is (are) homogenousely (and stably) fixed (applied, added and/or adsorbed) to the (stamped) (polyacrylamide) polymer hydrogel surfaces, for instance by putting (a large part of) the polyacrylamide (polymer) hydrogel in contact with a solution comprising the second (other) biomolecule (such as a peptide or protein or an ECM peptide or ECM protein); this solution comprising between about 1 and about 100 µg/ml, preferably between about 5 µg/ml and about 50 µg/ml of this second (other) biomolecule, preferably at least about 10, 15, 20, 30 or even 40 µg/ml.

Advantageously, the biomolecule can be (stably) fixed on the hydrogel of the present invention a few minutes after the polymerization (of the monomers), or hours, and even several days after the polymerization (of the monomers).

A related aspect of the invention is a polyacrylamide (polymer) hydrogel obtainable by the method of the present invention.

The present invention therefore relates to a polyacrylamide hydrogel comprising co-polymerized acrylamide, bisacrylamide (N,N'-Methylenebisacrylamide) and 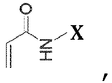 wherein the wt ratio of bisacrylamide to acrylamide is comprised between about 1% (w:w) and about 15 % (w:w), preferably between about 1.5% and about 12.5%, more preferably between about 3% and about 10% and/or the wt ratio of 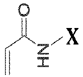 to acrylamide is comprised between about 5% and about 75%, preferably between about 10% and about 60%, more preferably between about 20% and about 50% and wherein X is a C₁-C₆ alkyl further comprising a function selected from the group consisting of hydroxyl, carboxy, amine and aldehyde.

Preferably, the alkyl group of this 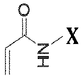 is an ethyl group.

Preferably, the alkyl (ethyl) group of this 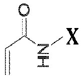 is substituted on the terminal carbon (meaning this molecule is a primary hydroxyl, a primary carboxy, a primary aldehyde or a primary amine).

Preferably, this 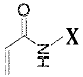 comprises exactly one function (on the terminal carbon of the alkyl (ethyl) group) selected from the group consisting of hydroxyl, carboxy, amine and aldehyde.

Preferably, the function (on the terminal carbon) of this 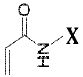 is a hydroxyl group.

More preferably, this 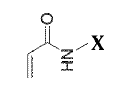 is N-hydroxyethylacrylamide (HEA).

Preferably this polyacrylamide hydrogel comprises between 1% (w:w) and 10% (w:w) of acrylamide.

Preferred hydrogels comprise thus between about 0.02% and about 1.25% of bisacrylamide, preferably between about 0.05 % and about 1% of bisacrylamide, more preferably between about 0.075% and about 0.75% of bisacrylamide, and from about 0.4% to about 4%, preferably from about 0.75% to about 2.5%, more preferably from about 1% to about 1.5% of 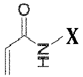 (such as HEA); all percentages are in weight ratios; i.e. weight monomer:total hydrogel weight.

Preferably this polyacrylamide hydrogel further comprises a first biomolecule (stably) fixed (applied, added and/or adsorbed) on its surface.

Advantageously, this first biomolecule is a protein, preferably selected from the group consisting of albumin, laminin, fibronectin, collagen I, collagen IV, streptavidin, tenascin, reelin, cadherin, (active) fragments and mixtures thereof, being more preferably laminin and/or fibronectin and/or ECM-related protein or fragments, or synthetic peptides (such as peptides comprising the RGD motif of fibronectin), or growth factors and chemokines (chemoattractants).

Preferably, the first biomolecule(s) is (are) non-randomly fixed (applied, added and/or adsorbed) to this polyacrylamide hydrogel and/or is (are) heterogeneously (stably) fixed (applied, added and/or adsorbed) to (the surface of) this polyacrylamide hydrogel.

Preferably, this polyacrylamide hydrogel further comprises a second (another) biomolecule (preferably a protein, more preferably albumin, such as bovine serum albumin) homogenously (and stably) fixed (applied, added and/or adsorbed) to (the surface of) this polyacrylamide hydrogel.

Advantageously, this polyacrylamide hydrogel further comprises cells, preferably animal (human) cells, such as (non human embryonic) stem cells fixed upon these biomolecules but also (primary and continuous) mammalian cells, (and even bacterial and yeast cells). Therefore, the polyacrylamide hydrogel according to the invention can be a biosensor or a biomaterial for the binding of cells.

A related aspect of the present invention concerns the use of this polyacrylamide hydrogel for the *in vitro* differentiation of animal (preferably mammalian, more preferably human non embryonic) stem cells.

Preferably, this animal cell is selected from the group consisting of endothelials cells (such as HUVECs), dermal keratocytes, fibroblasts, neuronal cell and a muscle cell, being more preferably neural cell or muscle cell.

### Examples

### Comparative Example

The inventors firstly synthesized polyacrylamide gels (acrylamide and bis-acrylamide) then placed them into a solution comprising 30-50 µg/ml of ECM peptides.
However, these ECM peptides were not adsorbed, nor retained by these gels.

The inventors then co-polymerized acrylamide gels (with bisacrylamide) with further amino-ethyl acrylamide or alkyl-cetone-derived acrylamide monomer (Diacetone acrylamide).

These gels were then placed into a solution comprising 30-50 µg/ml of ECM peptides, further in presence of glutaraldehyde in the case of the gel co-polymerized with amino-ethyl acrylamide.

Both gels have adsorbed some ECM proteins.

However, the gel that comprises glutaraldheyde has impaired mechanical properties. On the other hand, the gel co-polymerized with the alkyl-cetone acrylamide retained ECM proteins, but only for a limited time period of about 24 hours.

These gels were considered as of no interest, at least for cell culture, when involving long-time adhesion to stably fixed biomolecules.

### Example 1: Synthesis of hydroxy-PAAm hydrogels

Hydroxy-PAAm hydrogels were synthetized on a glass surface for the convenience of operating cell adhesion experiments. Circular glass coverslips of 25 mm in diameter were cleaned with 0.1 M NaOH (Sigma, Saint-Louis, MO) solution during 5 minutes and then rinsed abundantly (20 minutes under agitation) with deionized water. Cleaned coverslips were treated during one hour with 3-(Trimethoxysilyl)propyl acrylate (Sigma, Saint-Louis, MO) to promote strong adhesion between hydroxy-PAAm gel and glass and dried under a nitrogen flow. In a 15 mL Eppendorf tube, 500 µL acrylamide 40% w/w in HEPES (AAm, Sigma, Belgium), 250 to 1250 µL N,N'-methylenebisacrylamide 2% w/w in HEPES (BisAAm, Sigma, Saint-Louis, MO) and 1065 µL N-hydroxyethylacrylamide monomers (65 µg/mL in HEPES, Sigma, Saint-Louis, MO) were mixed and the desired volume of a solution of 4-(2-hydroxyethyl)-1-piperazineethanesulfonic acid 50 mM (HEPES, Sigma,Saint-Louis, MO) was added to reach a final volume of 5 mL. After degassing the mixture during 20 minutes under vacuum, the polymerization was started by adding 2.5 µL of N,N,N',N'-Tetramethylethylenediamine (TEMED, Sigma, Saint-Louis, MO) and 25 µL of ammonium persulfate solution (APS, Sigma, Saint-Louis, MO). A volume of 25 µL of this mixture was deposited on a 25 mm diameter glass coverslip and then a 22 mm diameter glass coverslip was placed upon the solution. After 30 minutes, polymerization was completed and the 22 mm diameter coverslip was gently removed to obtain a ∼65 µm thick hydroxy-PAAm hydrogel. Finally, hydroxy-PAAm hydrogels were washed three times in sterile deionized water and stored at 4°C in sterile Phosphate Buffer Saline (PBS).

### Example 2: Polydimethylsiloxane stamps

Micropatterns of different shapes (lines, circles, triangles, squares and rectangles of aspect ratios 1:2, 1:4 and 1:10), and various areas (1200-2500 µm2) were designed using Clewin software (WieWeb Software, Hengelo, The Netherlands). A chromium photomask (Toppan Photomask, Corbeil Essonnes, France) was generated to transfer micropatterns to a silicon master by dry reactive ion etching (FH Vorarlberg University of Applied Sciences, Microtechnology, Dornbirn, Austria). Microstamps were obtained by molding the silicon master with polydimethylsiloxane, PDMS, (Sylgard 184 Silicone Elastomer Kit, Dow Corning, Midland, MI) cured 3 h at 60°C. The silicon surface was passivated with a fluorosilane (tridecafluoro-1,1,2,2-tetrahydrooctyl-1-trichlorosilane, Gelest) for 30 min in vacuum to facilitate the peeling of the PDMS from the structured surface.

### Example 3: Mechanical measurement of hydroxy-PAAm gel stiffness

Hydroxy-PAAm hydrogels containing from 0.05 to 0.5 % w/w bis-AAm were synthesized.

Dynamic Mechanical Analysis (Mettler Toledo DMA/SDTA 861e, Switzerland) in compression was undertaken on circular cylindrical samples of diameter varying between 15 and 20 mm and height varying between 6,5 and 10 mm. Samples were sandwiched between two parallel plates and an oscillating strain of maximum amplitude of 10% was applied. The stress needed to deform the cylindrical samples (n=5) was measured over a frequency range of 0,1-10 Hz. During compression testing, a settling time of approximately one minute was used to achieve a stable measurement of the storage modulus E' at each frequency.

### Example 4 decoupled control of hydrogel stiffness and micropattern geometry

ECM-protein micropatterns were created on hydroxy-PAAm hydrogels by microcontact printing (µCP). ECM proteins were transferred to predesigned regions of flat surfaces by using elastomeric stamps obtained from the molding of microstructured silicon wafers with polydimethylsiloxane (PDMS).

During the first step, PDMS stamps were incubated with an ECM-protein solution at room temperature. After one hour, stamps were dried under a nitrogen flow and placed in conformal contact with hydroxy-PAAm surfaces at room temperature during one hour to obtain a homogeneous transfer of protein micropatterns on the soft substrates. After stamping, PDMS stamps were gently removed and stamped hydroxy-PAAm surfaces were passivated overnight at 4°C with bovine serum albumin (BSA) to block the non-printed surface areas. Finally, cells were seeded onto micropatterned hydroxy-PAAm surfaces and cells that did not bind specifically to the micropatterns were washed away.

Stamped ECM-proteins were labeled with fluorescein isothiocyanate (FITC) to clearly observe protein micropatterns by fluorescence microscopy (epifluorescence and/or confocal and/or Total Internal Reflection Fluorescence modes). Several patterns of arbitrary sizes and shapes were obtained, independently of the gel stiffness (2.5, 15 and 40 kPa).

Imaging of the gel surface with fluorescently labeled laminin (LM; an ECM protein) showed a uniformity of coverage and a constant amount of protein bound to both softest and stiffest gels.

A closer examination of the LM micropatterns using high-magnification objectives showed that protein microstamping on hydroxy-PAAm hydrogels results in sharp edges. The deep contrast between microprinted and BSA-passivated zones demonstrated the high affinity of hydroxy-PAAm hydrogels to form hydrogen-bonding interactions with LM and the efficient protein anti-adhesive ability of BSA passivated areas.

The inventors further analyzed the stability of ECM micropatterns by treating stamped hydroxy-PAAm hydrogels of varying stiffnesses (E = 2.5, 15 and 40 kPa) under UV sterilization for 20 min. or by incubating them in culture medium for 8 days. The shapes and the fluorescent intensity of protein micropatterns did not change significantly after UV exposure or culture medium incubation, demonstrating the high stability of ECM-proteins micropatterns on hydroxy-PAAm hydrogels over the wide range of gel rigidities.

### Example 5: Decoupled control of hydrogel stiffness and cell-ligand density

Hydroxy-PAAm substrates were printed with microstamps putting into contact of solutions of increasing concentrations of LM (10, 20 and 30 µg/mL), then dried. The inventors obtained a profile of pixel intensity, which indicated a deep contrast between printed and passivated zones and a constant pixel gray value over the patterns, demonstrating a high fidelity of the procedure, even at low ECM protein concentration.

The inventors have then observed the surface density for three bound-proteins (LM, FN and Streptavidin; SV) of concentrations ranging from 5 to 50 µg/mL by fluorescence microscopy under the same conditions of exposition and acquisition. The mean pixel gray value of the whole image was quantified and normalized by the ratio of the printed area to the total image area. In these settings, the fluorescence intensity of LM and FN was found to be linear as a function of the protein concentration up to 20 µg/mL before to reach an intensity threshold, indicating a saturation of bound proteins, whereas a linear dependence of fluorescence intensity on SV concentration was observed for the whole range of concentrations. Interestingly, the inventors measured no significant dependence of LM cell ligand density on the stiffness of hydroxy-PAAm hydrogels. This quantitative method enables therefore the decoupled control of the ligand surface density for a large range of biomolecules in a simple and efficient way.

### Example 6: Multi-ligand labeling and gradient of ECM proteins

FN and LM inked stamps were sequentially brought into conformal contact with a flat hydroxy-PAAm substrate for 1 hour to produce dual-labeled patterns, whereas a third stamp inked with collagen was used to obtain a multicomponent pattern. A solution of BSA was pipetted onto both surfaces over the protein stamped areas in a blocking step.

The dual labeled hydroxy-PAAm substrate was observed with a fluorescence microscope and excited under the FITC filter (excitation wavelength 482±35 nm) and the TRITC filter (excitation wavelength 537±50 nm), whereas the multicomponent labeled substrate was observed also under a DAPI filter (excitation wavelength 377±50 nm). All microprinted proteins fluoresced only under their respective filters, indicating a successful and selective transfer of multiple proteins on the same substrate.

The fluorescence signal for LM stamped across FN lines or for Collagen stamped across LM and FN lines, in accordance with their pre-designed stamp localization, and the labeling is continuous and undiminished at the overlay areas.

These observations demonstrated that hydroxy-PAAm hydrogels retain sufficient binding capacity, following immobilization of a first or a second protein, to permit additional protein immobilization.

Thus, whereas conventional functionalization techniques on soft hydrogels are mostly limited to generation of mono or dual component surfaces, microcontact printing on hydroxy-PAAm hydrogels enables easy preparation of multicomponent surface patterns.

Previous studies have reported that graded distributions of proteins are pivotal for understanding mechanotransduction and signaling processes, such as morphogenesis, cell migration or axon guidance. Interestingly, the high affinity of hydroxy-PAAm hydrogels for biomolecules allowed the inventors to generate immobilized protein gradients by employing consecutive microstamping steps.

### Example 7 Cell adhesion and viability of micropatterned hydroxy-PAAm Hydrogels

Primary Human Umbilical Vein Endothelial Cells (HUVECs) were plated on various geometries of FN micropatterns (circles, triangles, squares and triangles) deposited on hydrogels with varying rigidities. After complete spreading, HUVECs were fixed in paraformaldehyde, permeabilized with Triton X100, and then labeled to observe the nucleus, actin filaments, and vinculin-containing focal adhesions. Hydroxy-PAAm hydrogels are shown by the inventors to be functionally competent to support cell surface interactions. Staining for actin filaments, a specific component of the cell cytoskeleton, revealed that HUVECs spread well on the wide range of pattern geometries and remain strongly confined to FN micropatterns, regardless the matrix stiffness. The inventors conclude that the quality of the well-organized arrays of endothelial cells obtained on microprinted hydroxy-PAAm hydrogels demonstrates the potential of this technique, including for automated high-throughput assays for cell biology and for the screening of new pharmacological agents.

The inventors next investigated the toxicity of hydroxy-PAAm hydrogels by quantifying the viability of HUVECs plated on homogeneously coated and microprinted soft hydroxy-PAAm hydrogels for 1, 2 and 3 days in culture by using a live/dead viability cytotoxicity fluorescence assay to determine the percentage of viable cells by counting the number of living cells and dead cells with image analysis software. No significant difference in viability was observed between homogeneously coated and patterned hydrogels. Indeed, even if the viability on homogeneous FN coating was observed to be the highest, over ∼80% of HUVECs plated on micropatterned soft hydroxy-PAAm hydrogels maintained their viability for three days.

Endothelial cell proliferation was then measured through multiple cell counts on homogeneous coated 25 kPa hydroxy-PAAm gels and 500 kPa substrates after one and seven days in culture. Endothelial cells proliferated more quickly on 'stiff' (500 kPa) compared to 'soft' (25 kPa) substrates.

These results indicated that cells remained viable and proliferated across the whole range of stiffnesses, further demonstrating that this method provides the appropriate microenvironment for cellular investigation.

### Example 8 Cytoskeletal and nuclear remodelling respond to substrate stiffness

Hydroxy-PAAm hydrogels of three levels of rigidity (2.5, 8.5 and 25 kPa) were coated with FN to ensure a specific cellular adhesion. In order to discriminate the effect of substrate stiffness from that of ligand density, the FN concentration was held constant (50 µg/mL) over the range of stiffnesses, as observed by fluorescence microscopy.

However, previous studies have shown that many cell types, including endothelial cells, show large variations of the spreading area as a function of the substrate stiffness on which they adhere. Indeed, fibroblasts have been observed to spread three times more on 10 kPa gels (mean cell area ∼1500 µm2), than on 2 kPa gels (mean cell area ∼500 µm2).

In order to overcome the stiffness effect on cell spreading, individuals HUVECs were plated on rectangular (aspect ratio 1:4) FN-coated micropatterns of 1200 µm2. This strategy ensured to control both spreading area and geometry of HUVECs, regardless the stiffness of the microenvironment.

By using the ease of manipulating mechanical properties, biofunctionalization and spatial organization of proteins, hydroxy-PAAm hydrogels allow to overcome mechanotransduction complexity in order to identify clearly the relative effect of physico-chemical properties of the cell matrix.

After 24 hours in culture, HUVECs were fixed and stained to determine whether the organization of focal adhesion protein vinculin, actin filaments and the nucleus are dependent on the matrix stiffness.

HUVECs plated on the lower stiffness hydrogel had a low density of actin fibers, small focal adhesions distributed at the cell periphery and a round nucleus. In contrary, cells plated on higher stiffness hydrogel had thicker and straight actin fibers, larger vinculin-containing focal adhesion sites and a deformed nucleus. The quantification of focal adhesion sites indicated that the mean adhesion area per cell increased from 74±7 µm² on 2.5 kPa substrates to 86±7 µm² on 25 kPa substrates. Interestingly, the nuclear aspect ratio increased from ∼1.6 to ∼2.1 and the projected nuclear area underwent a decrease of ∼29% as the substrate stiffness increased. Therefore, the substrate stiffness has a profound effect on the cytoskeletal organization of endothelial cells and can impact on the nucleus.

Endothelial cells adjust their internal stiffness to match that of the substrate on which they spread. The response of endothelial cells to increased substrate stiffness in the range from 2.5 kPa to 25 kPa involved a reorganization of the actin cytoskeleton into a more organized system of filaments bundles and contractile stress fibers.

## Claims

1. A method for producing a polyacrylamide hydrogel comprising the steps of mixing, in a liquid solution, monomers of acrylamide, bisacrylamide and N-hydroxyethylacrylamide and an agent causing the co-polymerization of the said monomers.

2. The method of claim 1 further comprising the step of fixing a peptide to the polymer.

3. The method of claim 2, wherein the peptide is fixed by microcontact printing, preferably at a pressure of about 100 Pa to about 1 000 Pa.

4. A polyacrylamide hydrogel comprising co-polymerized acrylamide, bisacrylamide and N-hydroxyethylacrylamide, wherein the wt ratio of bisacrylamide to acrylamide is comprised between 1% and 15% and the wt ratio of N-hydroxyethylacrylamide to acrylamide is comprised between 20% and 50%.

5. The polyacrylamide hydrogel of claim 4 comprising between 1% (w:w) and 10% (w:w) of acrylamide.

6. The polyacrylamide hydrogel of claims 4 or 5, further comprising a first biomolecule fixed at the surface of the said polyacrylamide hydrogel.

7. The polyacrylamide hydrogel of claim 6, wherein the first biomolecule is a protein, preferably selected from the group consisting of albumin, laminin, fibronectin, collagen I, collagen IV, streptavidin, tenascin, reelin, cadherin, integrins, chemokines, antibodies and fragments thereof, being more preferably laminin and/or fibronectin.

8. The polyacrylamide hydrogel of claim 6 or 7, wherein the first biomolecule is fixed to the surface of the said hydrogel from a solution comprising between 1 µg/ml and 100 µg/ml of the said first biomolecule.

9. The polyacrylamide hydrogel of claims 6 or 7, wherein the first biomolecule is heterogeneously fixed to the said polyacrylamide hydrogel.

10. The polyacrylamide hydrogel of claim 9, wherein the first biomolecule is fixed on a surface of between about 500 µm² and between about 5000 µm².

11. The polyacrylamide hydrogel according to any of the preceding claims 6 to 10, further comprising a second biomolecule homogenously fixed to the said polyacrylamide hydrogel.

12. The polyacrylamide hydrogel according to any of the preceding claims 6 to 11, further comprising animal cells.

13. Use of the polyacrylamide hydrogel of claim 12 as biosensor.

14. Use of the polyacrylamide hydrogel according to any of the preceding claims 6 to 12, for the *in vitro* culture of animal cells.

15. **Use** of the polyacrylamide hydrogel according to any of the preceding claims 6 to 12, for the *in vitro* differentiation of animal stem cells.
